# EUROPEAN PATENT APPLICATION

(11) **EP 3 043 301 A1**
(43) Date of publication of application: **13.07.2016**
(21) Application number: 15202733.0
(22) Date of filing: 24.12.2015
(51) Int. Cl.: G06Q 10/10, G06Q 50/22

(54) **ELECTRONIC DEVICE AND METHOD FOR PROVIDING WORKOUT SERVICE IN ELECTRONIC DEVICE**

(30) Priority: 06.01.2015 KR 20150001290
(71) Applicant: Samsung Electronics Co., Ltd, Gyeonggi-do 16677 (KR)
(72) Inventor: KIM, Jeong Ja, Gyeonggi-do 16695 (KR); KIM, Jee Young, Gyeonggi-do 16512 (KR); KIM, Ji Eun, Gyeonggi-do 16517 (KR); SON, Tae Hwan, Gyeonggi-do 16697 (KR); SHIN, Hee Seung, Seoul 01042 (KR); YU, Eun Young, Seoul 04717 (KR); A HONG, Yeon, Gyeonggi-do 14984 (KR); MIN, Kyung Sub, Gyeonggi-do 13525 (KR)
(74) Representative: Jacobs, Bart

(57) **Abstract**

Disclosed is electronic device including an input module that receives a user instruction to select a workout program and a weekly workout day or a workout date of the workout program and a control module that establishes a schedule of the workout program according to the total number of workout days and the weekly workout day or the workout date of the workout program.

## Description

### BACKGROUND

### 1. Field of the Disclosure

The present disclosure relates to methods for providing proper workout services to users.

### 2. Description of the Related Art

There has recently been a growing interest in health, and consequently, in workouts for maintaining fitness. Therefore, electronic devices have offered workout services which systematically manage workouts for users. For example, electronic devices have provided workout applications that recommend proper workouts and plan workout schedules for users.

However, since electronic devices in the prior art uniformly recommend workouts to users without considering each user's schedule, the workout services of the prior art have been inconvenient for users and have caused users to miss workouts. As such, there is a need in the art for a workout service that considers user's schedules, thereby allowing the users to more consistently perform planned workouts.

### SUMMARY

Aspects of the present disclosure are to address at least the above-mentioned problems and/or disadvantages and to provide at least the advantages described below. Accordingly, an aspect of the present disclosure is to provide a method for providing a workout service having a workout schedule that considers a user's schedule and guides the user to perform an incomplete workout if the user misses a work out.

In accordance with an aspect of the present disclosure, an electronic device, includes an input module that receives a user instruction to select a workout program and a weekly workout day or a workout date of the workout program, and a control module that establishes a schedule of the workout program according to a total number of workout days and the weekly workout day or the workout date of the workout program.

In accordance with another aspect of the present disclosure, a method for providing a workout service in an electronic device includes receiving a user instruction to select a workout program, receiving a user instruction to select a weekly workout day or a workout date of the workout program, and establishing a schedule of the workout program according to the total number of workout days and the weekly workout day or the workout date of the workout program.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 illustrates a configuration of an electronic device according to embodiments of the present disclosure;
FIGS. 2A, 2B, and 2C are screens illustrating user interfaces (UIs) displayed on a display according to embodiments of the present disclosure;
FIGS. 3A and 3B are screens illustrating UIs displayed on a display according to embodiments of the present disclosure;
FIGS. 4A and 4B are screens illustrating UIs displayed on a display according to embodiments of the present disclosure;
FIGS. 5A, 5B, 5C, and 5D are screens illustrating UIs displayed on a display according to embodiments of the present disclosure;
FIGS. 6A, 6B, and 6C are screens illustrating UIs displayed on a display according to embodiments of the present disclosure;
FIG. 7 is a screen illustrating a UI displayed on a display according to embodiments of the present disclosure;
FIGS. 8A, 8B, and 8C are screens illustrating UIs displayed on a display according to embodiments of the present disclosure; and
FIG. 9 illustrates a method for providing a workout service in an electronic device according to embodiments of the present disclosure.

### DETAILED DESCRIPTION OF EMBODIMENTS OF THE DISCLOSURE

Hereinafter, the present disclosure is described with reference to the accompanying drawings. Various modifications are possible in embodiments of the present disclosure and embodiments are illustrated in drawings and related detailed descriptions are listed. However, the present disclosure is not limited thereto, and it is understood that all modifications and/or, equivalents and substitutes within the scope and technical range of the present disclosure are included. In the drawings, like reference numerals refer to like elements, and a description of well known functions or configurations will be omitted for the sake of clarity and conciseness.

As used herein, the expressions "include" and "comprise" or "may include" and "may comprise" indicate disclosed functions, operations, or existence of elements but do not exclude one or more additional functions, operations or elements. Also, it should be further understood that the terms "include" and "comprise" or "have" used herein specify the presence of disclosed features, integers, operations, elements, components, or combinations thereof but do not preclude the presence or addition of one or more other features, integers, operations, elements, components, or combinations thereof.

As used herein, the meaning of the expression "or" includes any or all combinations of the words connected by the term "or". For instance, the expression "A or B" may include A, B, or both A and B.

The expressions such as "1st", "2nd", "first", or "second" used herein refers to various elements of embodiments of the present disclosure, but do not limit the corresponding elements. For instance, such expressions do not limit the order and/or priority of the corresponding elements. The expressions may be used to distinguish one element from another element. For instance, both "a first user device" and "a second user device" indicate a user device but indicate different user devices from each other. For example, a first component may be referred to as a second component and vice versa without departing from the scope of the present disclosure.

It will be understood that when an element is referred to as being "connected" or "coupled" to another element, the element can be directly connected or coupled to the other element or intervening elements may be present. In contrast, when an element is referred to as being "directly connected" or "directly coupled" to another element, it should be understood that there are no intervening elements between the element and the another element.

Terms used in this specification are used to describe specified embodiments of the present disclosure and are not intended to limit the scope of the present disclosure. The terms of a singular form may include plural forms unless otherwise specified.

Unless otherwise defined herein, all the terms used herein, which include technical or scientific terms, may have the same meaning that is generally understood by a person skilled in the art.

It will be further understood that terms, which are defined in a dictionary and commonly used, should also be interpreted as is customary in the relevant related art and not in an idealized or overly formal detect unless expressly so defined herein.

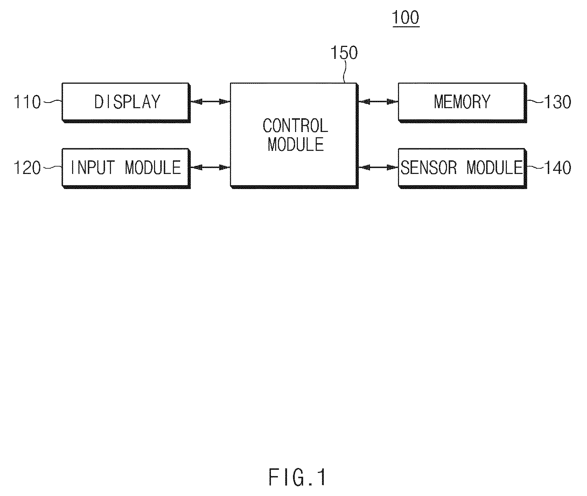

FIG. 1 illustrates a configuration of an electronic device according to embodiments of the present disclosure.

Referring to FIG. 1, an electronic device 100 includes a display 110, an input module 120, a memory 130, a sensor module 140, and a control module 150.

The display 110 displays a UI according to execution of a workout service application. According to embodiments of the present disclosure, the display 110 displays a UI including various types of objects such as text, an image, and an icon. The electronic device 100 receives a user instruction and provides necessary information to a user through the UI displayed on the display 110.

The input module 120 receives a user instruction to select at least one of a workout program, a workout start date, a workout end date, a workout date, a weekly workout day, the total number of workout days, and the total number of workout weeks. According to an embodiment of the present disclosure, the weekly workout day is selected on a weekly basis and the workout day is selected on a monthly basis.

According to an embodiment of the present disclosure, an item that is selected by the user may be changed according to a type of a workout program selected by the user. For example, if the user selects a first workout program, a workout start date and a weekly workout day of the first workout program is selected by the user. If the user selects a second workout program, a workout start date, a weekly workout day, and the total number of workout days of the second workout program may be selected by the user.

According to an embodiment of the present disclosure, the input module 120 includes at least one of a touch screen and a touch pad which operates by a touch input of the user, a keypad or a keyboard which has various function, numeral, special, and character keys, a remote controller, a mouse, a motion sensor which recognizes motion of the user, and a voice sensor which recognizes a voice of the user.

The memory 130 stores user information such as name, gender, height, and weight, a workout program schedule, a schedule progress state, a schedule completion state, workout records, and rewards according to schedule completionworkout program schedule, such as for each workout program. According to an embodiment of the present disclosure, the memory 130 stores information associated with a workout program in which a schedule is ended or a workout program that is partially completed as well as a workout program which is currently in progress. According to an embodiment of the present disclosure, information associated with a workout program in which a schedule is ended or a workout program that is partially completed may be deleted according to a user instruction.

The sensor module 140 senses workout records according to physical activities or a state of the user. For example, the sensor module 140 senses the number of steps, the number of revolutions of bicycle pedals, a workout distance, a change in altitude, a heart rate of the user, and speed. According to an embodiment of the present disclosure, the sensor module 140 includes various types of sensors, such as a gyro sensor, an acceleration sensor, an altitude sensor, a heart rate sensor, and a global positioning system (GPS) sensor, for measuring workout records and a state of the user.

The control module 150 controls an overall operation of the electronic device 100. The control module 150 controls each of the display 110, the input module 120, the memory 130, and the sensor module 140 to establish a workout program schedule and provide a workout service to the user according to embodiments of the present disclosure. For example, when a user instruction to execute an application is entered through the input module 120, the control module 150 executes the application stored in the memory 130 and provides a workout service.

According to an embodiment of the present disclosure, the control module 150 establishes a workout program schedule according to the total number of workout days and a weekly workout date of a workout program. The schedule of the workout program includes, for example, a workout date, workout contents, the entire workout time, detailed workout contents, and a detailed workout time.

The control module 150 controls the display 110 to display a UI for receiving a workout program schedule and a user's schedule. According to an embodiment of the present disclosure, the UI for receiving the user's schedule includes objects that receive a workout start date, a workout end date, a workout date, a weekly workout day, the total number of workout days, and the total number of workout weeks.

The control module 150 controls the display 110 to display a UI for viewing the entire workout program schedule before or after the workout program is started. The UI for viewing the entire schedule of the workout program includes information about whether a day is a workout or rest day, a workout date, workout contents, or a workout time with respect to the number of workout days. When a workout program is started, an object indicating whether each schedule is completed may be displayed along with the schedule on the display 110.

According to an embodiment of the present disclosure, if the workout program in which the schedule is established is started, the control module 150 controls the display 110 to display a schedule corresponding to the present day's date or a schedule for the nearest future. For example, the display 110 displays the schedule corresponding to the present day's date or the schedule for the nearest future on an application dashboard or a menu screen indicating the present day's schedule.

According to an embodiment of the present disclosure, if an incomplete schedule is present among schedules of a workout program, the control module 150 reestablishes the incomplete schedule at a date when there is no workout schedule. For example, if the schedule of the workout program is set weekly to Monday, Wednesday, and Friday and a workout scheduled for Friday of the present week is not completed, a schedule for Friday is reset to Saturday of the present week.

According to an embodiment of the present disclosure, if an incomplete schedule is present among schedules of a workout program, the control module 150 may change workout contents or a workout time of another workout schedule. For example, if the schedule of the workout program is set weekly to Monday, Wednesday, and Friday and a workout scheduled for Wednesday of the present week is not completed, a workout time of a schedule for Friday of the present week is changed from 30 minutes to one hour. The incomplete schedule refers to when the user does not work out at all or only partially completes the workout contents or workout time.

According to an embodiment of the present disclosure, if an incomplete schedule is present among schedules of a workout program, the control module 150 controls the display 110 to display at least one of a workout program, a workout date, the number of workout days, workout contents, the remaining number of workout days, the entire workout time, detailed workout contents, or a detailed workout time in the incomplete schedule. The control module 150 further controls the display 110 to display a UI for starting the incomplete schedule.

According to an embodiment of the present disclosure, the control module 150 controls the display 110 to display a UI indicating a schedule progress state of a workout program on a daily, weekly, or monthly basis. A UI indicating a daily schedule progress state includes, for example, information about whether a schedule is completed, a workout date, and workout records such as a workout distance and a workout time. A UI indicating a weekly schedule progress state includes, for example, a schedule completion rate of a corresponding week, the total workout records of the corresponding week, and a workout date and work records for each schedule. A UI indicating a monthly schedule progress state includes, for example, the total number of schedules of a corresponding month, the number of completed schedules of the corresponding month, the number of incomplete schedules of the corresponding month, a schedule completion rate of the corresponding month, the total workout records of the corresponding month, and a workout date and workout records for each schedule.

According to an embodiment of the present disclosure, the control module 150 offers rewards on a daily, weekly, monthly, or entire schedule basis according to whether a workout program schedule is completed. For example, the control module 150 offers rewards if a schedule is completed at a predetermined rate or more a week, a month, or in the entire schedule. The reward may include, for example, a graphic image can be displayed in the display 110, a coupon of a tangible item, such as food, electronic goods, clothes, and the like, a ticket (or a coupon) of an intangible item corresponding to a particular facility (e.g., a water park ticket, a movie ticket, an amusement park ticket, a fitness center ticket, and the like), and the like. According to an embodiment of the present disclosure, the control module 150 controls the display 110 to display a UI indicating a list of rewards that may be offered. If specific rewards are offered to the user, an offered date of the corresponding rewards is displayed. The offered rewards are distinguishably displayed from rewards that are not offered.

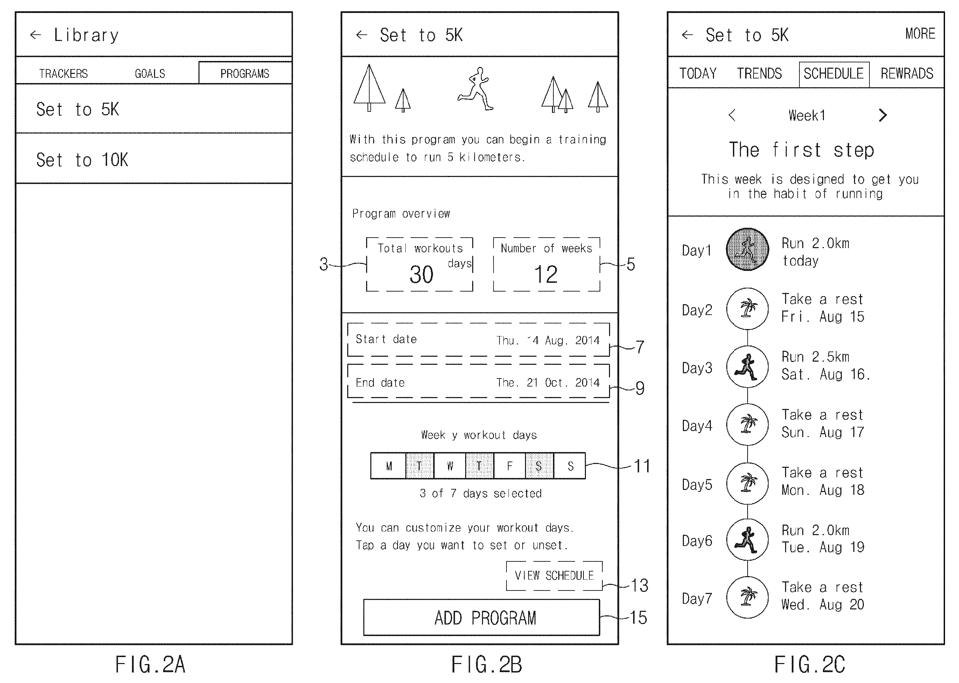

FIGS. 2A, 2B, and 2C are screens illustrating UIs displayed on a display according to embodiments of the present disclosure.

Referring to FIG. 2A, a display 110 of FIG. 1 displays a UI for selecting a workout program. Referring to FIG. 2A, the display 110 displays a menu for selecting a running program of a 5-kilometer course and a 10-kilometer course. According to an embodiment of the present disclosure, the display 110 displays a menu for selecting various workout programs according to a type of a workout such as walking, running, and cycling, difficulty of the workout such as a beginning or an intermediate level, or a purpose of the workout such as dieting, cardiopulmonary function strengthening, or muscle strength strengthening. If a user instruction to select the 5-kilometer course is entered in FIG. 2A, as shown in FIG. 2B, the display 110 displays a UI for receiving a user's schedule.

Referring to FIG. 2B, the UI which receives the user's schedule includes objects for selecting the total number of workout days 3, the total number of workout weeks 5, a workout start date 7, a workout end date 9, and weekly workout days 11. The user may enter an item value associated with a corresponding object by touching the corresponding object included in the UI. An additional UI for receiving an item value may be provided according to an item type. In one example, the user touches alphabets denoting Monday to Sunday included in the object for selecting the weekly workout days 11 to select or release the selection of the corresponding weekly workout days. In another example, if the user selects the object for selecting the workout start date 7, a calendar for receiving a workout start date is displayed.

According to an embodiment of the present disclosure, the total number of workout days 3 or the total number of workout weeks 5 may be set as an item which should not be selected. The workout end date 9 may be determined according to the total number of workout days 3, the workout start date 7, and the weekly workout days 11. If the total number of workout days 3, the total number of workout weeks 5, the workout start date 7, the workout end date 9, and the weekly workout days 11 are completed, a control module 150 of FIG. 1 establishes a workout program schedule. If the total number of workout days 3, the total number of workout weeks 5, the workout start date 7, the workout end date 9, and the weekly workout days 11 are completed, a workout program start object 15 is activated. If the user selects the workout program start object 15, a schedule of the corresponding workout program progresses.

Referring to FIG. 2B, the UI that receives the user's schedule includes an object 13 for viewing the entire workout program schedule. If the user selects the object 13, as shown in FIG. 2C, the schedule of the workout program is displayed. For example, information about whether today is a workout day or a rest day, a workout date, workout contents, or a workout time is displayed with respect to the number of workout days. Therefore, before starting a specific workout program, the user may verify detailed information such as the workout date, the workout contents, and the workout time.

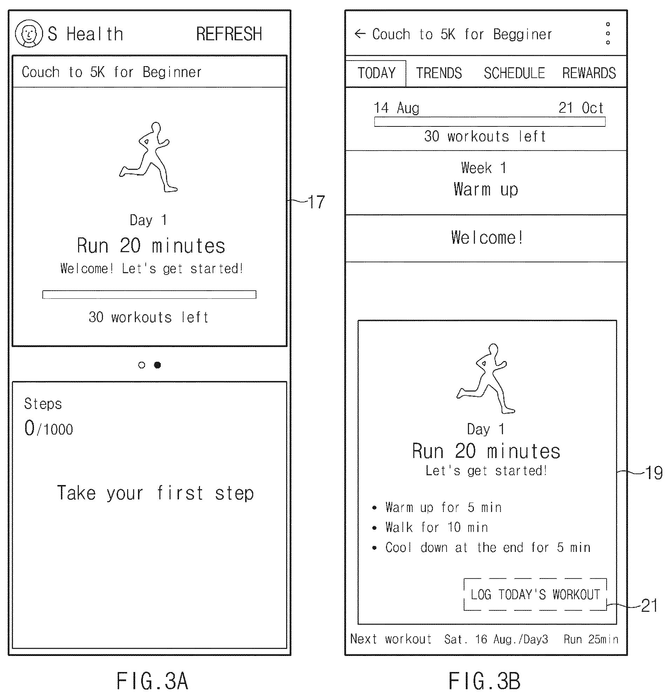

FIGS. 3A and 3B are screens illustrating UIs displayed on a display according to embodiments of the present disclosure.

Referring to FIG. 3A, if a workout program is started, information 17 about a schedule corresponding to the present day date or a schedule for the nearest future is displayed on an application dashboard. The schedule information 17 displayed on the dash board includes a workout program, the number of workout days, workout contents, a workout time, and the remaining number of workout days.

Referring to FIG. 3B, if the workout program is started, information 19 about the schedule corresponding to the present day date or the schedule for the nearest future may be displayed on the present day schedule menu of the application. The schedule information 19 displayed on the present day schedule menu includes the number of workout days, workout contents, a workout time, detailed workout contents, and a detailed workout time. If the schedule (or a workout) corresponding to the present day date is present, an object 21 for performing the present day schedule is additionally displayed. If the user selects a region that displays the schedule information 17 on a UI screen shown in FIG. 3A, the UI screen shown in FIG. 3A is changed to a UI screen shown in FIG. 3B.

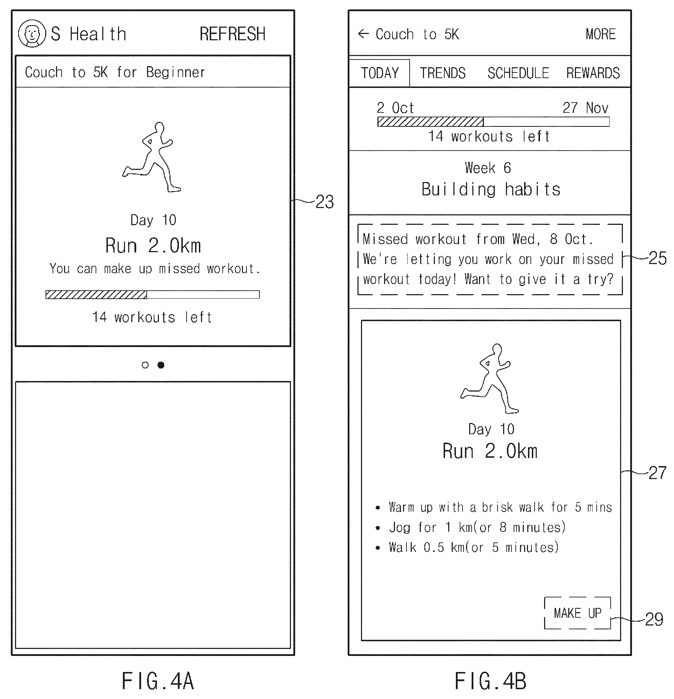

FIGS. 4A and 4B are screens illustrating UIs displayed on a display according to embodiments of the present disclosure.

Referring to FIG. 4A, if an incomplete schedule is present among schedules of a workout program, incomplete schedule information 23 is displayed on an application dashboard. The incomplete schedule information 23 displayed on the dashboard includes a workout program, the number of workout days, workout contents, a workout time, and the remaining number of workout days.

Referring to FIG. 4B, if an incomplete schedule is present among the schedules of the workout program, an object 25 indicating that the incomplete schedule is present and incomplete schedule information 27 is displayed on the present day schedule menu of the application. The object 25 indicating that the incomplete schedule is present includes a date of the incomplete schedule and text for guiding a user to complete the corresponding incomplete schedule. The incomplete schedule information 27 displayed on the present day schedule menu includes the number of workout days, workout contents, a workout time, detailed workout contents, and a detailed workout time. According to an embodiment of the present disclosure, an object 29 for performing an incomplete schedule is additionally displayed on the incomplete schedule information 27 displayed on the present day schedule menu. If the user selects a region which displays the schedule information 23 on a UI screen shown in FIG. 4A, the UI screen shown in FIG. 4A is changed to the UI screen shown in FIG. 4B.

If the incomplete schedule is present, a user may be informed of the incomplete schedule through various paths. An opportunity to complete the incomplete schedule may be provided to the user to guide the user to work out continuously.

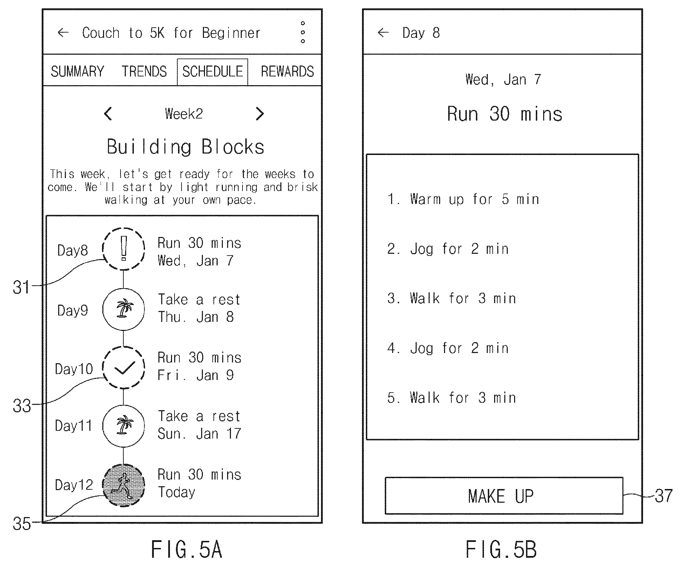

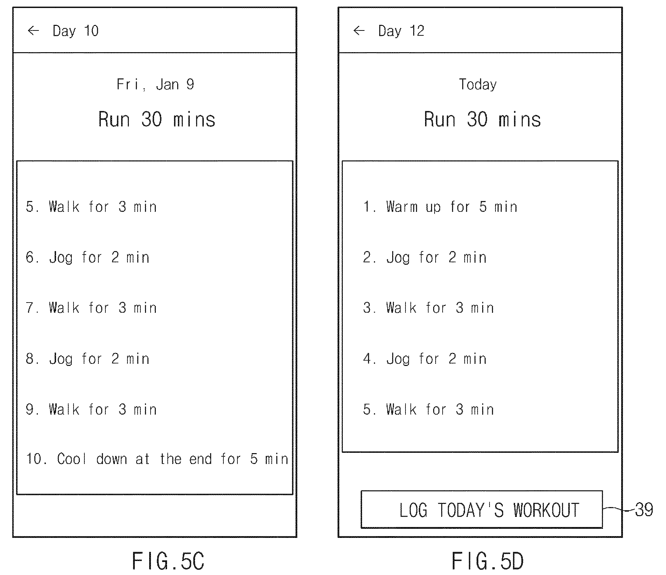

FIGS. 5A, 5B, 5C, and 5D are screens illustrating UIs displayed on a display according to embodiments of the present disclosure.

Referring to FIG. 5A, after a workout program is started, a UI indicating a schedule of the workout program is displayed on an application schedule menu. The UI indicating the schedule of the workout program displays, for example, information about whether the present day is a workout day or a rest day, a workout date, workout contents, or a workout time with respect to the number of workout days. The UI indicating the schedule of the workout program includes objects 31 and 33 indicating whether the schedule is completed. In one example, in case of an incomplete schedule, the object 31 such as an exclamation mark shape may be displayed. In another example, in case of a completed schedule, the object 33 such as a check mark shape is displayed. The UI indicating the schedule of the workout program includes an object 35 indicating the present day schedule. For example, the object 35 of a running person is displayed on the present day schedule.

According to an embodiment of the present disclosure, if each workout schedule included in the UI indicating the schedule of the workout program is selected according to a user instruction, a UI indicating detailed information of the corresponding schedule is displayed. If a schedule for Day 8 is selected among schedules displayed on FIG. 5A, as shown in FIG. 5B, a UI indicating detailed information of the schedule for Day 8 is displayed. Referring to FIG. 5B, a workout date, workout contents, a workout time, detailed workout contents, and a detailed workout time in the schedule for Day 8 is displayed. If the schedule for Day 8 is an incomplete schedule, an object 37 for starting the incomplete schedule (or a workout) is also displayed. If a schedule for Day 10 is selected among the schedules displayed in shown in FIG. 5A, as shown in FIG. 5C, a UI indicating detailed information of the schedule for Day 10 is displayed.

Referring to FIG. 5C, a workout date, workout contents, a workout time, detailed workout contents, and a detailed workout time in the schedule for Day 10 are displayed. If the schedule for Day 10 is a completed schedule, workout records such as a workout time or a workout distance of a user are displayed together. If a schedule for Day 12 is selected among the schedules shown in FIG. 5A, as shown in FIG. 5D, a UI indicating detailed information of the schedule for Day 12 is displayed. Referring to FIG. 5D, a workout date, workout contents, a workout time, detailed workout contents, and a detailed workout time in the schedule for Day 12 are displayed. If the schedule for Day 12 is the present day schedule, an object 39 for starting the present day schedule or workout is displayed.

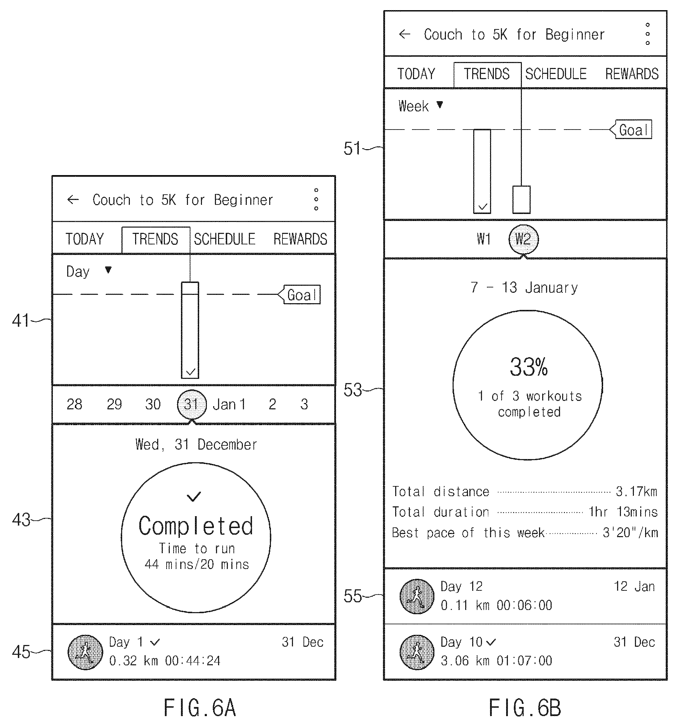

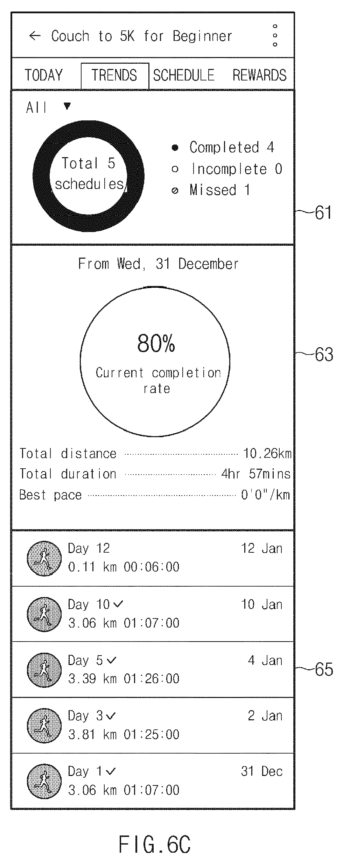

FIGS. 6A, 6B, and 6C are screens illustrating UIs displayed on a display according to embodiments of the present disclosure.

Referring to FIG. 6A, a display 110 of FIG. 1 displays a UI indicating a daily schedule progress state. The UI includes, for example, a region 41 which displays workout records of a corresponding date as a graph, a region 43 which displays information indicating whether a schedule of the corresponding date is completed, a workout date, and workout records such as a workout distance or a workout time, and a region 45 which displays a workout date and workout records for each schedule.

Referring to FIG. 6B, the display 110 displays a UI indicating a weekly schedule progress state. The UI includes, for example, a region 51 which displays a schedule completion state of a corresponding week as a graph, a region 53 which displays a schedule completion rate of the corresponding week and the total workout records of the corresponding week, and a region 55 which displays a workout date and workout records for each schedule.

Referring to FIG. 6C, the display 110 displays a UI indicating a monthly schedule progress state. The UI includes, for example, a region 61 which displays the total number of schedules of the corresponding month, the number of completed schedules of the corresponding month, the number of incomplete schedules of the corresponding month, and the number of missed schedules of the corresponding month, a region 63 which displays a schedule completion rate of the corresponding month and the total workout records of the corresponding month, and a region 65 which displays a workout date and workout records for each schedule.

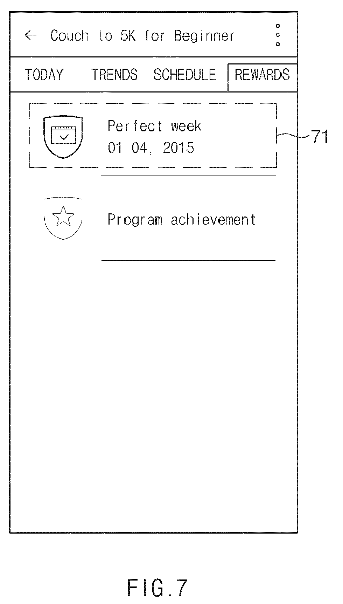

FIG. 7 is a screen illustrating a UI displayed on a display according to embodiments of the present disclosure.

Referring to FIG. 7, a display 110 of FIG. 1 displays a UI indicating a list of rewards which may be obtained. The UI indicating the rewards list prominently displays obtained rewards 71 and rewards which are not obtained. If a user obtains specific rewards, a payment date of the corresponding rewards is displayed. Rewards are paid to the user according to a schedule progress state of a workout program to guide the user to continuously work out.

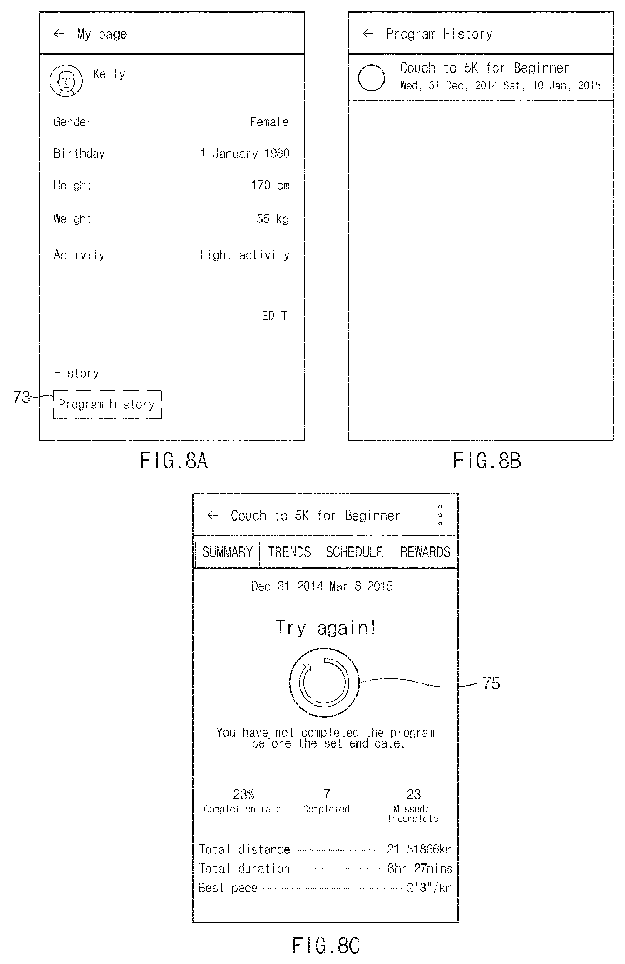

FIGS. 8A, 8B, and 8C are screens illustrating UIs displayed on a display according to embodiments of the present disclosure.

Referring to FIG. 8A, a display 110 of FIG. 1 displays a UI indicating user information and a workout program history. The UI indicating the user information includes a name, photo, gender, birthday, height, weight and activity of a user. The user information is entered by the user and is stored in the memory 130 of FIG. 1. The user information may be changed by the user. If the user selects an object 73 indicating a workout program history, as shown in FIG. 8B, a list of workout programs previously performed by the user is displayed.

Referring to FIG. 8B, the workout program list includes a type of a workout program and a period when the workout program is performed. If the user selects the workout program list, as shown in FIG. 8C, detailed information for the selected workout program is displayed. Referring to FIG. 8C, the period when the workout program is performed, the number of times that schedules are completed, the number of times that schedules are missed or incomplete, a schedule completion rate, and the total workout records are displayed. An object 75 for re-attempting the corresponding workout program according to a schedule completion state is also displayed. If the user selects the object 75, as shown in FIG. 2B, a UI that receives a user's schedule is displayed.

The following are aspects according to embodiments of the present disclosure:
The electronic device includes an input module that receives a user instruction to select a workout program and a weekly workout day or a workout date of the workout program and a control module that establishes a schedule of the workout program according to the total number of workout days and the weekly workout day or the workout date of the workout program

The schedule of the workout program includes a workout date, workout contents, the entire workout time, detailed workout contents, and a detailed workout time.

The input module receives a user instruction to select a workout day on a weekly basis or on a monthly basis.

The electronic device further includes a display configured to display a UI for selecting the weekly workout day or the workout date of the workout program.

The electronic device further includes a display configured to display at least one of a workout date, the number of workout days, workout contents, the entire workout time, detailed workout contents, and a detailed workout time in a schedule to be performed today, if the schedule to be performed today is present among schedules of the workout program.

The control module reestablishes an incomplete schedule at a date when there is no workout schedule, if the incomplete schedule is present among schedules of the workout program.

The control module changes workout contents or a workout time of another workout schedule, if an incomplete schedule is present among schedules of the workout program.
the electronic device further includes a display configured to display at least one of a workout program, a workout date, the number of workout days, workout contents, the remaining number of workout days, the entire workout time, detailed workout contents, and a detailed workout time in an incomplete schedule, if the incomplete schedule is present among schedules of the workout program.

The electronic device further includes a display configured to display a UI for starting an incomplete schedule, if the incomplete schedule is present among schedules of the workout program.

The control module offers rewards on a daily, weekly, monthly, or entire schedule basis according to whether the schedule of the workout program is completed.

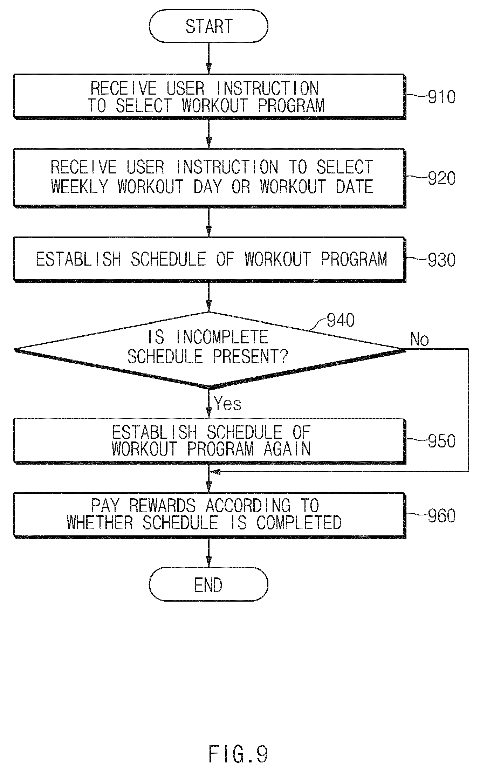

FIG. 9 illustrates a method for providing a workout service in an electronic device according to embodiments of the present disclosure. The steps in FIG. 9 are processed in an electronic device 100 shown in FIG. 1. Therefore, although some steps may be omitted in FIG. 9, contents described in reference to the electronic device 100 may be applied to the following steps in FIG. 9.

Referring to FIG. 9, in step 910, the electronic device 100 receives a user instruction to select a workout program. According to an embodiment of the present disclosure, the electronic device 100 displays a menu for selecting various workout programs according to a type of a workout such as walking, running, or cycling, difficulty of the workout such as a beginning or an intermediate level, or a purpose of the workout such as dieting, cardiopulmonary function strengthening, or muscle strength strengthening on a display 110 of FIG. 1. A user of the electronic device 100 may select one of workout programs displayed on the display 110.

In step 920, the electronic device 100 receives a user instruction to select a weekly workout day or a workout date of the selected workout program. According to an embodiment of the present disclosure, the electronic device 100 displays a UI for selecting at least one of the total number of workout days, the total number of workout weeks, a workout start date, a workout end date, or weekly workout days. The user selects a weekly workout day or a workout date through the UI displayed on the display 110. The weekly workout day is selected on a weekly basis and the workout date is selected on a monthly basis.

In step 930, the electronic device 100 establishes a schedule of the workout program according to the total number of workout days and the weekly workout day or the workout date of the workout program. The schedule of the workout program includes, for example, a workout date, workout contents, the entire workout time, detailed workout contents, and a detailed workout time.

In step 940, the electronic device 100 determines whether an incomplete schedule is present among schedules of the workout program. The incomplete schedule refers to a schedule when the user does not work out at all or the user works out but does not complete the workout contents.

If it is determined that the incomplete schedule is present, in step 950, the electronic device 100 reestablishes a schedule of the workout program, such as on a date when there is no workout schedule. For example, if a schedule of the workout program is set weekly to Monday, Wednesday, and Friday and if a workout scheduled for Friday of the present week is not completed, a schedule for Friday is reset to Saturday of the present week. According to an embodiment of the present disclosure, if it is determined that the incomplete schedule is present, the electronic device 100 changes workout contents or a workout time of another workout schedule. For example, if the schedule of the workout program is set weekly to Monday, Wednesday, and Friday and if a workout scheduled for Wednesday of the present week is not completed, a workout time of a schedule for Friday of the present week is changed from 30 minutes to one hour.

According to an embodiment of the present disclosure, if it is determined that the incomplete schedule is present, the electronic device 100 controls the display 110 to display at least one of a workout program, a workout date, the number of workout days, workout contents, the remaining number of workout days, the entire workout time, detailed workout contents, or a detailed workout time in the incomplete schedule. According to an embodiment of the present disclosure, if the incomplete schedule is present among the schedules of the workout program, the electronic device 100 controls the display 110 to display a UI for starting the incomplete schedule. The electronic device 110 displays the incomplete schedule through various paths or the UI to guide the user to complete the incomplete schedule.

In step 960, the electronic device 100 offers rewards on a daily, weekly, monthly, or entire schedule basis according to whether the schedule of the workout program is completed. For example, if the schedule is completed at predetermined rates or more a week, a month, or in the entire schedule, the electronic device 100 offers rewards. According to an embodiment of the present disclosure, the electronic device 100 displays a UI indicating a list of rewards which may be offered on the display 110. If specific rewards are offered to the user, an offered date of the corresponding rewards is displayed. The offered rewards are distinguishably displayed from rewards which are not offered.

The following are additional aspects according to embodiments of the present disclosure:
If a workout program in which a schedule is established is started, the electronic device 100 controls the display 110 to display a schedule corresponding to the present day date or a schedule for the nearest future. For example, the display 110 displays the schedule corresponding to the present day date or the nearest upcoming schedule on an application dashboard or a menu screen indicating the present day schedule. The electronic device 100 displays an incomplete schedule through various paths or a UI to guide the user to continuously work out.

The electronic device 100 displays a UI indicating a schedule progress state on the display 110. The electronic device 100 controls the display 110 to display a schedule progress state of a workout program on a daily, weekly, or monthly basis. A UI indicating a daily schedule progress state includes, for example, information about whether a schedule is completed, a workout date, and workout records. A UI indicating a weekly schedule progress state includes, for example, a schedule completion rate of a corresponding week, the total workout records of the corresponding week, a workout date for each schedule, and workout records for each schedule. A UI indicating a monthly schedule progress state includes, for example, the total number of schedules of a corresponding month, the number of completed schedules of the corresponding month, the number of missed or incomplete schedules of the corresponding month, a schedule completion rate of the corresponding month, the total workout records of the corresponding month, a workout date for each schedule, and workout records for each schedule.

A method for providing a workout service in an electronic device includes receiving a user instruction to select a workout program, receiving a weekly workout day or a workout date of the workout program, and establishing a schedule of the workout program according to the total number of workout days and the weekly workout day or the workout date of the workout program.

The schedule of the workout program includes a workout date, workout contents, the entire workout time, detailed workout contents, and a detailed workout time.

The user instruction to select the weekly workout day or the workout date of the workout program is received on a weekly basis or on a monthly basis.

The method further includes displaying a UI, for selecting the weekly workout day or the workout date of the workout program, on a display.

The method further includes displaying at least one of a workout date, the number of workout days, workout contents, the entire workout time, detailed workout contents, or a detailed workout time in a schedule to be performed today on a display, if the schedule to be performed today is present among schedules of the workout program.

The method further includes establishing an incomplete schedule again at a date when there is no workout schedule, if the incomplete schedule is present among schedules of the workout program.

The method further includes changing workout contents or a workout time of another workout schedule, if an incomplete schedule is present among schedules of the workout program.

The method further includes displaying at least one of a workout program, a workout date, the number of workout days, workout contents, the remaining number of workout days, the entire workout time, detailed workout contents, and a detailed workout time in an incomplete schedule on a display, if the incomplete schedule is present among schedules of the workout program.

The method further includes displaying a UI, for starting an incomplete schedule, on a display, if the incomplete schedule is present among schedules of the workout program.

The method further includes paying rewards on a daily, weekly, monthly, or entire schedule basis according to whether the schedule of the workout program is completed.

The terminology "module" used herein may mean, for example, a unit including one of hardware, software, and firmware or two or more combinations thereof. The terminology "module" may be interchangeably used with, for example, terminologies "unit", "logic", "logical block", "component", or "circuit". The "module" may be a minimum unit of an integrated component or a part thereof. The "module" may be a minimum unit performing one or more functions or a part thereof. The "module" may be mechanically or electronically implemented. For example, the "module" may include at least one of an application-specific integrated circuit (ASIC) chip, fieldprogrammable gate arrays (FPGAs), or a programmable-logic device, which is well known or will be developed in the future, for performing certain operations.

According to embodiments of the present disclosure, at least a part of a device such as modules or the functions or a method may be implemented with, for example, instructions stored in a computer-readable storage media which has a program module. If the instructions are executed by a processor, the one or more processors may perform functions corresponding to the instructions. The computer-readable storage media may be, for example, a memory 130 of FIG. 1.

The computer-readable storage media may include a hard disc, a floppy disk, magnetic media such as a magnetic tape, optical media such as a compact disc read only memory (CD-ROM), a digital versatile disc (DVD), magneto-optical media such as a floptical disk, and a hardware device such as a ROM, a random access memory (RAM), or a flash memory. The program instructions may include not only mechanical codes compiled by a compiler but also high-level language codes which may be executed by a computer using an interpreter and the like. The above-mentioned hardware device may be configured to operate as one or more software modules to perform operations according to embodiments of the present disclosure, and vice versa.

Modules or program modules according to embodiments of the present disclosure may include at least one or more of the above-mentioned components, some of the above-mentioned components may be omitted, or other additional components may be further included therein. Operations executed by modules, program modules, or other elements may be executed by a successive method, a parallel method, a repeated method, or a heuristic method. Also, some of the operations may be executed in a different order or may be omitted, and other operations may be added.

According to embodiments of the present disclosure, the electronic device may allow the user to more effectively perform a planned workout and to continuously work out according to the planned schedule by providing a workout schedule in consideration of a schedule of the user.

Embodiments of the present disclosure described and shown in the drawings are provided as examples to describe technical content and help understanding but do not limit the scope of the present disclosure. Accordingly, it should be interpreted that besides the embodiments listed herein, all modifications or modified forms derived based on the technical ideas of the present disclosure are included in the scope of the present disclosure as defined in the claims, and their equivalents.

While the present disclosure has been shown and described with reference to certain embodiments thereof, it will be understood by those skilled in the art that changes in form and details may be made therein without departing from the scope of the present disclosure as defined by the appended claims.

## Claims

1. An electronic device, comprising:
an input module that receives a user instruction to select a workout program and a weekly workout day or a workout date of the workout program; and
a control module that establishes a schedule of the workout program according to a total number of workout days and the weekly workout day or the workout date of the workout program.

2. The electronic device of claim 1, wherein the schedule of the workout program comprises a workout date, workout contents, a total workout time, detailed workout contents, and a detailed workout time.

3. The electronic device of claim 1 or 2, wherein the input module receives the user instruction to select the weekly workout day on a weekly basis or to select the workout date on a monthly basis.

4. The electronic device of any of the previous claims, further comprising:
a display that displays a user interface for selecting the weekly workout day or the workout date of the workout program.

5. The electronic device of any of the previous claims, further comprising:
a display that displays at least one of the workout date, workout contents, the entire workout time, detailed workout contents and a detailed workout time, in a schedule to be performed on a present day, if the schedule to be performed on the present day is present among schedules of the workout program.

6. The electronic device of any of the previous claims, wherein the control module reestablishes an incomplete schedule at a date when there is no workout schedule, if the incomplete schedule is present among schedules of the workout program.

7. The electronic device of any of the previous claims, wherein the control module changes workout contents or a workout time of another workout schedule, if an incomplete schedule is present among schedules of the workout program.

8. The electronic device of any of the previous claims, further comprising:
a display that displays at least one of the workout program, the workout date, the total number of workout days, workout contents, a remaining number of workout days, and a detailed workout time in an incomplete schedule, if the incomplete schedule is present among schedules of the workout program.

9. The electronic device of any of the previous claims, further comprising:
a display that displays a user interface for starting an incomplete schedule, if the incomplete schedule is present among schedules of the workout program.

10. The electronic device of any of the previous claims, wherein the control module offers rewards on a daily, weekly, monthly, or entire schedule basis according to whether the schedule of the workout program is completed.

11. A method for providing a workout service in an electronic device, the method comprising:
receiving a user instruction to select a workout program;
receiving a user instruction to select a weekly workout day or a workout date of the workout program; and
establishing a schedule of the workout program according to the total number of workout days and the weekly workout day or the workout date of the workout program.

12. The method of claim 11, wherein the schedule of the workout program comprises the workout date, workout contents, a total workout time, and a detailed workout time.

13. The method of claim 11 or 12, wherein the user instruction is received to select the weekly workout day on the weekly basis or to select the workout date on a monthly basis.

14. The method of any of the claims 11 - 13, further comprising:
displaying a user interface, for selecting the weekly workout day or the workout date of the workout program, on a display.

15. The method of any of the claims 11-14, further comprising:
displaying, on a display, at least one of the workout date, workout contents, the entire workout time, detailed workout contents and a detailed workout time, in a schedule to be performed on a present day, if the schedule to be performed on the present day is present among schedules of the workout program.
